**Europäisches Patentamt**

**European Patent Office**

**Office européen des brevets**

(19)

(11) Veröffentlichungsnummer : **0 028 203**
**B1**

(12)

# EUROPÄISCHE PATENTSCHRIFT

(45) Veröffentlichungstag der Patentschrift :
**15.12.82**

(21) Anmeldenummer : **80810279.2**

(22) Anmeldetag : **08.09.80**

(51) Int. Cl.³ : **B 01 J 14/00**, B 01 J 19/00,
C 07 B   1/00, C 07 B   3/00,
C 07 B 11/00, C 07 B 13/00,
C 07 B 29/00, C 07 C 76/02,
C 07 C113/00, C 08 F   2/00//
C07C79/10, F28C3/04

(54) **Verfahren zur Abführung der Reaktionswärme exothermer chemischer Reaktionen und Vorrichtung zur Durchführung des Verfahrens.**

(30) Priorität : **13.09.79 CH 8296/79**

(43) Veröffentlichungstag der Anmeldung :
**06.05.81 (Patentblatt 81/18)**

(45) Bekanntmachung des Hinweises auf die Patenterteilung : **15.12.82 Patentblatt 82/50**

(84) Benannte Vertragsstaaten :
**AT BE CH DE FR GB IT LI NL SE**

(56) Entgegenhaltungen :
**DE A 1 900 091**
**DE A 2 635 273**
**FR A 1 354 797**
**FR A 1 367 524**
**FR A 1 419 092**
**NL C 71 928**
**US A 2 739 174**
**US A 2 773 911**
**US A 3 086 849**
**US A 3 092 671**
**US A 3 178 481**

(73) Patentinhaber : **CIBA-GEIGY AG**
**Patentabteilung Postfach**
**CH-4002 Basel (CH)**

(72) Erfinder : **Altorfer, Fritz**
**Baselstrasse 31**
**CH-4132 Muttenz (CH)**

# Verfahren zur Abführung der Reaktionswärme exothermer chemischer Reaktionen und Vorrichtung zur Durchführung des Verfahrens

Die vorliegende Erfindung betrifft ein Verfahren zur Abführung der Reaktionswärme von in flüssiger Phase ablaufenden kontinuierlich geführten exothermen chemischen Reaktionen sowie eine Vorrichtung zur Durchführung dieses Verfahrens.

Die Abführung der Reaktionswärme von exothermen chemischen Reaktionen ist ein sehr wichtiges Problem in der chemischen Verfahrenstechnik. Im Falle von Reaktionen, die in flüssiger Phase ablaufen, d.h. bei denen im Reaktionsraum mindestens eine flüssige Phase vorhanden ist, sind verschiedene Lösungen dieses Problems bekannt. So kann in der Labortechnik beispielsweise der Reaktionsraum (das Reaktionsgefäss) in ein Kühlbad eingebracht werden, in dem sich ein flüssiges (z.B. Wasser, Oel, organische Kühlflüssigkeiten) oder festes (Eis, Trockeneis, Kältemischungen, z.B. aus Eis und Salzen) Kühlmittel befindet. Diese Art der Kühlung hat oft den Nachteil, dass sie schwer regulierbar ist, für kontinuierliche Prozesse häufig ungeeignet, zu wenig wirkungsvoll und unwirtschaftlich (z.B. Trockeneis) ist. Weiterhin ist es im Betriebsmassstab möglich, den Reaktionsraum mit Rohrschlangen oder einem Mantel zu umgeben, durch die eine Kühlflüssigkeit geleitet wird, z.B. Wasser, Kühlsole (Wasser mit Salzen, wie z.B. Natriumchlorid, Calciumchlorid, Magnesiumchlorid), organische Kühlflüssigkeiten, verflüssigte Gase, gegebenenfalls auch geschmolzene Feststoffe, wie flüssige Salze und Metalle. Wenn eine sehr intensive Kühlung erforderlich ist, können auch Kühlschlangen in das Innere des Reaktionsraumes geführt werden, die dann mit dem Kühlmittel beschickt werden. Als spezielle Kühlsysteme seien auch noch Verdunstungskühler und Rieselkühler erwähnt. Alle vorgenannten Kühlsysteme erfordern verhältnismässig aufwendige apparative Konstruktion, sind nicht für alle Zwecke wirksam genug oder sind im Betrieb unwirtschaftlich.

Es ist auch bekannt, zur Kühlung einer exothermen Reaktion ein verflüssigtes Inertgas (z.B. Stickstoff) direkt in das Reaktionsmedium einzuleiten. Diese Methode ist jedoch aufwendig und wenig wirtschaftlich, da das Gas nicht zurückgewonnen werden kann. Eine Kühlung kann auch einfach durch Zugabe von Eis oder Trockeneis direkt in das Reaktionsgemisch erreicht werden, doch ist in diesem Fall eine Regulierung der Temperatur schwierig. Ausserdem erfolgt bei Verwendung von Eis eine Verdünnung des Reaktionsmediums, was in vielen Fällen unerwünscht ist.

In der US-A 3 092 671 ist ein Verfahren zur kontinuierlichen Nitrierung in einem Schlaufenreaktor beschrieben, wobei die gesamte Reaktionsmischung nach Verlassen des Reaktionsraumes jeweils durch einen Wärmeaustauscher abgekühlt wird und ein Teil der so abgekühlten Reaktionsmischung zu Kühlzwecken in den Reaktionsraum zurückgeführt wird. Dass jeweils das gesamte Reaktionsgemisch abgekühlt werden muss, ist aus Gründen der Energiebilanz nicht vollständig befriedigend.

Ziel der vorliegenden Erfindung ist es, ein einfaches und wirtschaftliches Verfahren zur Abführung der Reaktionswärme von in flüssiger Phase ablaufenden, kontinuierlich geführten, exothermen chemischen Reaktionen zu finden, das die Nachteile der bekannten Verfahren nicht aufweist, das es erlaubt, das Volumen der Reaktionsapparatur möglichst klein zu halten und das vom Standpunkt der Betriebssicherheit Vorteile bringt.

Diese Ziele können überraschenderweise durch das erfindungsgemässe Verfahren erreicht werden, das dadurch gekennzeichnet ist, dass man eine gegenüber den Reaktionspartnern inerte, diese und die Reaktionsprodukte vorzugsweise nicht oder nur wenig lösende, mit dem Reaktionsmedium oder Teilen des Reaktionsmediums nicht mischbare Flüssigkeit in einen das Reaktionsmedium enthaltenden Reaktor einleitet, worin eine innige Durchmischung erfolgt, die entstandene Emulsion in die die Reaktionsprodukte enthaltende Phase und die erwärmte Inertflüssigkeit auftrennt, letztere abkühlt und wieder dem Reaktor zuführt, wobei die Inertflüssigkeit diesen Kreislauf beliebig oft durchlaufen kann.

Nach der Rückführung der Inertflüssigkeit in den Reaktor kann letztere wieder Reaktionswärme aufnehmen. Durch den ständigen Kreislauf der Inertflüssigkeit stellt sich eine gewünschte stationäre Temperatur im Reaktor ein.

Das erfindungsgemässe Verfahren ist zur Kühlung aller exothermen Reaktionen geeignet, die in flüssiger Phase ablaufen. Unter solchen Reaktionen sind selbstverständlich nicht nur Reaktionen in homogener flüssiger Phase zu verstehen, sondern alle Reaktionen, an denen mindestens eine flüssige Phase beteiligt ist. Dazu gehören auch Reaktionen in einem fest/flüssigen sowie in einem flüssig/gasförmigen System. Auch Reaktionen, an denen zwei flüssige Phasen beteiligt sind, können mit dem erfindungsgemässen Verfahren gekühlt werden, jedoch nur dann, wenn keine der beiden beteiligten Phasen mit der zur Kühlung verwendeten Inertflüssigkeit mischbar ist.

Alle exothermen Reaktionen der vorstehend definierten Art können mit Hilfe des erfindungsgemässen Verfahrens gekühlt werden, sofern die eingesetzte Inertflüssigkeit nicht an der Reaktion teilnimmt, vorzugsweise Ausgangsprodukte und Reaktionsprodukte nicht oder nur wenig löst und nicht mit der Reaktionsphase bzw. mit einer der Reaktionsphasen mischbar ist. Als Beispiele für Reaktionen, die auf diese Weise gekühlt werden können, seien exotherme Substitutions-, Oxidations-, Reduktions-, Additions-, Eliminierungs- oder Polymerisationsreaktionen genannt. Sehr gut eignet sich das erfindungsgemässe Verfahren zur Kühlung von exotermen Nitrierungen, Sulfonierungen, Diazotierungen, Hydrierungen und anderen Reduktionsreaktionen. Besonders bevorzugt wird das erfindungsgemässe Verfahren zur Wärmeabführung bei der Nitrierung von aromatischen Systemen eingesetzt.

Wie bereits erwähnt, kann als Inertflüssigkeit jede Flüssigkeit eingesetzt werden, die an der jeweiligen Reaktion nicht teilnimmt und die mit dem Reaktionsmedium (oder Teilen des Reaktionsmediums) nicht mischbar ist und in der die Ausgangsstoffe und Endprodukte vorzugsweise nicht oder nur wenig löslich sind. Je nach Intensität der Wärmetönung der zu kühlenden Reaktion bzw. je nach der Temperatur, bei der die Reaktion ablaufen soll, müssen Flüssigkeiten gewählt werden, deren Siedepunkt über jener Temperatur liegt, um zu verhindern, dass die Flüssigkeit zu sieden beginnt.

Vor dem Eintritt in den Reaktor wird die vom vorhergehenden Durchgang durch den Reaktor erwärmte Inertflüssigkeit gekühlt. Dies geschieht in einer üblichen Kühlvorrichtung, z.B. in einem üblichen Wärmeaustauscher. Die Temperatur, auf die die Flüssigkeit abgekühlt wird, richtet sich nach der Reaktion, die gekühlt werden soll und der Temperatur, die im Reaktor aufrechterhalten werden soll, sowie nach der Natur der Flüssigkeit selbst. Zu tiefe Temperaturen sind aus wirtschaftlichen Ueberlegungen nicht vorteilhaft (hohe Energiekosten). Die Temperaturen des Kühlmittels können daher in weiten Grenzen schwanken, etwa zwischen − 50° und + 50 °C, insbesondere zwischen − 20° und + 20 °C.

Eine weitere Möglichkeit, die gewünschte Temperatur im Reaktor zu beeinflussen, ist die Veränderung der Verweilzeit im Reaktor durch Variieren der Geschwindigkeit, mit der die Inertflüssigkeit durch den Kühlkreislauf zirkuliert. Soll bei einer gegebenen Temperatur der Inertflüssigkeit stärker gekühlt werden, kann die Menge derselben, die in den Reaktor eindosiert wird, erhöht werden. Auf diese Weise lässt sich im Reaktor die gewünschte Temperatur leicht einstellen.

Für Reaktionen in wässriger Phase kommen als Inertflüssigkeiten vorzugsweise mit Wasser nicht mischbare organische Flüssigkeiten, z.B. übliche mit Wasser nicht mischbare organische Lösungsmittel in Betracht. Dazu gehören u.a. aliphatische, cycloaliphatische oder aromatische Kohlenwasserstoffe, halogenierte aliphatische oder aromatische Kohlenwasserstoffe, mit Wasser nicht mischbare Alkohole, Ketone, Ester und Aether.

Von den aliphatischen Kohlenwasserstoffen seien vor allem Benzine verschiedenster Siedebereiche (Testbenzin, Lackbenzin, Naphtha usw.) und Paraffinkohlenwasserstoffe genannt. Die einzelnen flüssigen Kohlenwasserstoffe können natürlich auch in reiner Form eingesetzt werden.

Von den cycloaliphatischen Kohlenwasserstoffen eignen sich z.B. Cyclohexan, Cyclopentan, Methylcyclohexan, Tetralin, Dekalin und andere.

Auch gewisse Terpenkohlenwasserstoffe und Terpenoide können, sofern sie nicht mit Wasser mischbar sind, eingesetzt werden.

Als aromatische Kohlenwasserstoffe seien beispielsweise erwähnt : Benzol, Toluol, Xylol, Cumol, Cymol, Styrol sowie Mischungen davon.

Unter den halogenierten Kohlenwasserstoffen sind vor allem chlorierte Kohlenwasserstoffe zu erwähnen : Methylenchlorid, Chloroform, Tetrachlorkohlenstoff, 1,2-Dichloräthan, 1,1,2,2-Tetrachloräthan, 1,1,1-Trichloräthan, 1,1,2-Trichloräthan, Perchloräthylen, Propylenchlorid sowie Mischungen davon, ferner Mono-, Di- oder Trichlor- und -brombenzole.

Wasserunlösliche Alkohole, die als Kühlflüssigkeiten verwendet werden können, sind z.B. Cyclohexanol, Methylcyclohexanol sowie Fettalkohole mit längerer Kohlenwasserstoffkette.

Als wasserunlösliche Ketone seien z.B. erwähnt : Methyl-n-butylketon, Methylisobutylketon, Methyl-n(i)-amylketon, Aethylamylketon, Di-n-propylketon, Diisopropyl- und -butylketon, Mesityloxid, Cyclohexanon, Methylcyclohexanon, Dimethylcyclohexanon, Isophoron.

Als wasserunlösliche Ester seien beispielsweise erwähnt : n-Butylformiat, n-Propylacetat, n-Butylacetat, Isobutylacetat, n-Amylacetat, Isoamylacetat, Hexylacetat, Cyclohexylacetat, Benzylacetat sowie die entsprechenden Propionsäure- und Buttersäureester ; Butylglycolacetat, Aethyldiglycolacetat, Butyldiglycolacetat.

Von den wasserunlöslichen Aethern können beispielsweise verwendet werden : Diisopropyläther, Dibutyläther, Methyl-tert.-butyläther.

Darüberhinaus kann natürlich eine grosse Zahl von weiteren, mit Wasser nicht mischbaren Flüssigkeiten verwendet werden, soweit sie die oben angegebenen Bedingungen erfüllen.

Läuft umgekehrt eine Reaktion in einem organischen Lösungsmittel ab, so kann einerseits als Inertflüssigkeit eine organische Flüssigkeit, die mit dem Lösungsmittel der Reaktion nicht mischbar ist, oder andererseits Wasser verwendet werden, sofern das Lösungsmittel im Reaktor mit Wasser nicht mischbar ist. Statt Wasser können natürlich auch Salzlösungen verwendet werden, sofern sie auf die Reaktion keinen Einfluss nehmen.

Für die Funktionsfähigkeit des Verfahrens ist es erforderlich, dass zwischen dem Reaktionsmedium und der zur Kühlung dienenden Inertflüssigkeit eine innige Durchmischung stattfindet. Auf diese Weise wird die Fläche, durch die der Wärmeaustausch vonstatten geht, gross und der Wärmedurchgang findet durch ausserordentlich dünne Schichten statt. Dadurch wird auch eine möglichst kurze Austauschzeit erreicht.

Aus dieser Forderung ergibt sich, dass ein Reaktionsgefäss (Reaktor) verwendet werden muss, das eine derart innige Durchmischung gewährleisten kann. Im einfachsten Fall kann dazu ein üblicher Reaktionskessel mit Schnellrührer dienen. Bevorzugt sind aber Reaktoren, die eine noch bessere und innigere Durchmischung ermöglichen. Als derartige Reaktoren seien beispielsweise erwähnt : ein Zentrifugalpumpenreaktor, ein On-line-Mischer mit Förderpumpe, ein statischer Mischer mit Förderpumpe, eine Mischturbine oder ein Strahldüsenreaktor.

Die im Reaktor gebildete Emulsion von Reaktionsmedium und Inertflüssigkeit wird danach in einen Abscheider geführt, in dem die beiden Phasen getrennt werden, am einfachsten durch Absetzen der schwereren Phase. Es können natürlich auch andere Phasentrennapparaturen eingesetzt werden, z.B. ein Fliehkraftabscheider. Die die Reaktionsprodukte bzw. restlichen Ausgangsstoffe enthaltende Phase (Reaktionsgemisch) wird zu weiterer Aufarbeitung kontinuierlich abgezogen. Die Inertflüssigkeit wird durch einen Kühler (Wärmeaustauscher) geführt, in dem sie auf die gewünschte Temperatur abgekühlt wird und anschliessend in den Reaktor zurückgeführt, in dem sie wieder Reaktionswärme aufnehmen kann. Durch die beschriebene erfindungsgemässe Verfahrensführung wird also eine Kühlschlaufe mit einer weitgespannten Wärmeaufnahmekapazität geschaffen.

Falls es aufgrund der Gegebenheiten (z.B. Reaktionsgeschwindigkeit, Verweilzeit im Reaktor, optimale Reaktionstemperatur usw.) bei der jeweils durchgeführten Reaktion wünschenswert oder erforderlich ist, kann nach dem eigentlichen Reaktor und vor der Trennung der Emulsion eine Nachreaktionszone vorgesehen werden, um ein vollständiges Ausreagieren und damit eine Erhöhung der Ausbeute zu erreichen. Dies kann bei langsamer ablaufenden Reaktionen oder/und bei kurzen Verweilzeiten im eigentlichen Reaktor angebracht sein. Die Nachreaktionszone ist vorzugsweise als Reaktionsrohr ausgebildet, das bei Bedarf auch gekült werden kann, also mit einem Wärmeaustauscher versehen sein kann, falls die jeweilige Reaktion es erfordert oder dadurch die Ausbeute und Qualität des Endproduktes günstig beeinflusst wird.

Die Vorteile des erfindungsgemässen Kühlverfahrens gegenüber bekannten Verfahren bestehen vor allem darin, dass das Volumen der verwendeten Reaktionsapparatur klein gehalten werden kann, dass die Betriebssicherheit erheblich erhöht wird (intensive Kühlung direkt im Reaktionsgefäss, keine Gefahr der Ueberhitzung) und dass die Wirtschaftlichkeit durch Verringerung des Energieaufwandes erheblich erhöht werden kann.

Die vorliegende Erfindung betrifft auch eine Vorrichtung zur Durchführung des beschriebenen Verfahrens, gekennzeichnet durch

a) einen Reaktor mit Zuleitungen für die Ausgangsprodukte und gegebenenfalls für Lösungsmittel, und mit einer Zuleitung für die Inertflüssigkeit ;

b) einen Abscheider, der durch eine Leitung mit dem Reaktor verbunden ist und in dem die Produktelösung von der Inertflüssigkeit getrennt wird und

c) eine mit einem Wärmeaustauscher versehene Rückführungsleitung, die den Abscheider mit dem Reaktor verbindet.

Zwischen dem Reaktor und dem Abscheider kann noch eine Nachreaktionszone eingeschaltet sein. Vorzugsweise ist die Leitung zwischen Reaktor und Abscheider als Nachreaktionsrohr ausgebildet. Gegebenenfalls kann letzteres noch mit einem Wärmeaustauscher versehen sein.

Als Reaktor kann in der erfindungsgemässen Vorrichtung ein üblicher Reaktionskessel mit Schnellrührer dienen. Vorzugsweise wird als Reaktor jedoch eine Zentrifugalpumpe, ein On-line-Mischer mit Förderpumpe, ein statischer Mischer mit Förderpumpe, eine Mischturbine oder ein Stradhldüsenreaktor verwendet. Besonders vorteilhaft ist die Verwendung einer Zentrifugalpumpe als Reaktor.

Als Abscheider wird vorteilhaft ein solcher eingesetzt, in dem sich die beiden Phasen aufgrund der Schwerkraft trennen. Es Können natürlich auch andere Phasentrennapparaturen eingesetzt werden, z.B. ein Fliehkraftabscheider.

Die allgemeine Funktionsweise der erfindungsgemässen Vorrichtung ist der vorgängigen Beschreibung des erfindungsgemässen Verfahrens leicht zu entnehmen.

Die Zeichnungsfigur zeigt eine besondere Ausführungsform der erfindungsgemässen Vorrichtung in schematischer Darstellung. Darin bedeuten die einzelnen Positionsziffern folgendes :

Pos.

1 und 3 Vorratsgefässe für Ausgangsprodukte, Lösungen von Ausgangsprodukten bzw. Lösungsmittel

2 und 4 Dosierpumpen

5 Reaktor, vorzugsweise Zentrifugalpumpenreaktor

6 Reaktionsrohr für Nachreaktion

7 Abscheider

8 Vorlage für die die Reaktionsprodukte enthaltende Phase

9, 9a Wärmeaustauscher

10 Leitung für Reaktionsmischung/Inertflüssigkeits-Suspension

11 Ableitung für die die Reaktionsprodukte enthaltende Phase

12 Rückführungsleitung für die Inertflüssigkeit

13 Dosierventil für Inertflüssigkeit

Zur Durchführung des Verfahrens mit Hilfe der in der Zeichnungsfigur gezeigten Vorrichtung wird die zur Kühlung benutzte Inertflüssigkeit von der Leitung 12 über den Reaktor 5, die Leitung 10 und den Abscheider 7 zurück in die Leitung 12 im Kreislauf geführt (« Kühlschlaufe »). Aus den Vorratsgefässen 1 und 3 werden über die Dosierpumpen 2 und 4 die Reaktionspartner und das Lösungsmittel, in dem die Reaktion stattfindet, in den Reaktor eindosiert. Beispielsweise enthält, wenn eine Nitrierung durchgeführt werden soll, das Vorratsgefäss 1 Salpetersäure, das Vorratsgefäss 2 die zu nitrierende Verbindung, z.B. eine aromatische carbocyclische Verbindung, gegebenenfalls gelöst in Schwefelsäure. Im Reaktor 5, der in dieser Ausführungsform vorzugsweise eine Zentrifugalpumpe ist, und in dem die Reaktion abläuft, wird das Reaktionsmedium innig mit der in den Reaktor einströmenden Inertflüssigkeit vermischt, welche die entstehende Reaktionswärme aufnimmt. Nach der Verweilzeit im Reaktor (z.B. eine bis einige Sekunden) wird die gebildete Emulsion aus Reaktionslösung und Inertflüssigkeit in das Reaktionsrohr 6 gedrückt, in dem die Reaktion (z.B. die Nitrierung) zu Ende geführt wird. Das Reaktionsrohr 6 kann, falls aus irgend einem Grund erforderlich, auch gekühlt werden. Durch das Verbindungsrohr 10 strömt die Emulsion in den Abscheider 7, worin sich die Phasen trennen. Die sich absetzende Phase, welche die Endprodukte enthält (z.B. Nitriersäure, in der die nitrierten Produkte gelöst sind, wird durch die Leitung 11 in eine Vorlage 8 abgelassen. Die überstehende Inertflüssigkeit (es wird hier eine Inertflüssigkeit verwendet, deren spezifisches Gewicht kleiner ist als das der bevorzugt wässrigen Reaktionslösung) gelangt an seinem oberen Ende in das Rohr 12, welches von einem Kühler (Wärmeaustauscher) umgeben ist. Die Inertflüssigkeit wird durch diesen Kühler auf die gewünschte Temperatur abgekühlt und gelangt, gegebenenfalls nach Regulierung der Durchflussmenge durch das Ventil 13 zurück in den Reaktor. Die Inertflüssigkeit strömt auf diese Weise kontinuierlich durch den Reaktor und nimmt so die Reaktionswärme auf und gibt sie an den Wärmeaustauscher 9 ab.

Die nachfolgenden Beispiele dienen der weiteren Erläuterung des erfindungsgemässen Verfahrens.

Beispiel 1

Nitrierung von 4-Acetamidoanisol mit Hilfe der in der Zeichnungsfigur dargestellten Vorrichtung

Im Vorratsgefäss 1 werden 1 900 ml einer 1,579 molaren Lösung von 4-Acetamidoanisol in 93 %-iger Schwefelsäure vorgelegt. Im Vorratsgefäss 3 legt man 220 ml 62,58 %-ige Salpetersäure vor. Die Kühlschlaufe wird mit 2 200 ml Benzin mit einem Siedebereich von 110°-140 °C gefüllt. Man setzt die Zentrifugalpumpe 5 in Betrieb und bringt die Kühlmedium-temperaturen der Wärmeaustauscher auf folgende Werte : Wärmeaustauscher 9 : − 5 °C. Wärmeaustauscher 9a : − 15 °C. Sobald das Benzin in der Schlaufe eine Temperatur von − 2 °C erreicht hat, werden die beiden Reaktanden mit den Dosierpumpen 2 und 4 in den Reaktor eindosiert. Der Durchsatz an Salpetersäure beträgt 11 ml (0,15 Mol)/Min., jener an 4-Acetamidoanisol in Schwefelsäure 95 ml (0,15 Mol)/Min. Nach ca. 4 Minuten stellen sich stationäre Bedingungen ein. Eine erwünschte Reaktoraustrittstemperatur von 25 °C ist dann erreicht. Zu hohe oder zu tiefe Reaktionstemperatur lässt sich problemlos über die Kühlung des Wärmeaustauschers 9 regulieren. Der Versuch dauert 20 Minuten. Die erhaltene wässrige Produktelösung, die aus dem Abschneider 7 abgezogen wird, wird hernach auf Eiswasser ausgetragen und das ausgefallene Produkt filtriert. Ausbeute : 558 g mit einem Gehalt von 98,7 % 4-Acetamido-2-nitroanisol. Dies entspricht einer Ausbeute von 87,4 % der Theorie.

Beispiel 2

Die Nitrierung von Beispiel 1 wird wiederholt, jedoch wird die Kühlschlaufe mit 2 200 ml Dichlorbenzol gefüllt. Der Durchsatz an Salpetersäure beträgt 12,8 ml/Min., jener der Acetamidoanisollösung in Schwefelsäure 97,5 ml/Min., der totale Durchsatz an Flüssigkeit durch die Schlaufe beträgt ca. 1 300 ml/Min., das Reaktionsvolumen in der Pumpe beträgt 37 ml, jenes im Nachreaktionsrohr 14 ml. Die Verweilzeit in der Pumpe beträgt 1,7 Sekunden, jene im Nachreaktionsrohr 0,7 Sekunden, stationäre Bedingungen stellen sich nach 4-6 Minuten ein. Die Aufarbeitung des Reaktionsproduktes erfolgt wie im Beispiel 1. Man erhält auf diese Weise pro Minute 28,4 g 4-Acetamido-2-nitroanisol mit einem Gehalt von 98 %, entsprechend einer Ausbeute von 88,4 % der Theorie.

Beispiel 3

Nach den beiden in den Beispielen 1 und 2 beschrieben Verfahren wird 4-Propionamidoanisol nitriert. Man erhält auf diese Weise 4-Propionamido-2-nitroanisol in einer Ausbeute von ca. 90 % der Theorie.

Beispiel 4

Nach den beiden in den Beispielen 1 und 2 beschriebenen Verfahren wird 4-Acetamido-äthoxybenzol nitriert. Man erhält auf diese Weise 4-Acetamido-2-nitro-äthoxybenzol in einer Ausbeute von ca. 90 % der Theorie.

Beispiel 5

Nach den beiden in den Beispielen 1 und 2 beschriebenen Verfahren wird 2,5-Dichlor-acetanilid nitriert. Man erhält auf diese Weise 2,5-Dichlor-4-nitro-acetanilid in einer Ausbeute von ca. 90 % der Theorie.

Beispiel 6

Nach den beiden in den Beispielen 1 und 2 beschriebenen Verfahren wird 4-Chlorbenzoesäure nitriert. Man erhält auf diese Weise 4-Chlor-3-nitrobenzoesäure in einer Ausbeute von ca. 97 % der Theorie.

Beispiel 7

Nach den beiden in den Beispielen 1 und 2 beschriebenen Verfahren wird Benzoesäure nitriert. Man erhält auf diese Weise 3-Nitrobenzoesäure in einer Ausbeute von ca. 76 % der Theorie.

Beispiel 8

Im Vorratsgefäss 1 werden 986 ml einer 1,5 molaren Lösung von m-Toluidin in 98 %-iger Schwefelsäure vorgelegt. Im Vorratsgefäss 3 legt man 251 ml 40 %-ige Nitrosylschwefelsäure vor. Die Kühlschlaufe wird mit 2 200 ml Benzin mit einem Siedebereich von 110°-140 °C gefüllt. Man setzt die Zentrifugalpumpe 5 in Betrieb und bringt die Kühlmediumtemperaturen der Wärmeaustauscher auf folgende Werte : Wärmeaustauscher 9 : 10 °C, Wärmeaustauscher 9a : Raumtemperatur. Sobald das Benzin in der Schlaufe eine Temperatur von 18 °C erreicht hat, werden beide Reaktanden mit den Dosierpumpen 2 und 4 in den Reaktor eindosiert. Der Durchsatz an Nitrosylschwefelsäure beträgt 12,6 ml (0,075 Mol)/Min., jener an m-Toluidin in Schwefelsäure 49,3 ml (0,075 Mol)/Min. Nach ca. 2 Minuten stellen sich stationäre Bedingungen ein. Eine erwünschte Reaktionstemperatur von 20 °C ist dann erreicht. Eine zu hohe oder zu tiefe Reaktionstemperatur lässt sich problemlos über die Kühlung des Wärmeaustauschers 9 regulieren. Der Versuch dauert 20 Minuten. Den erhaltenen Diazokörper, der aus dem Abscheider 7 abgezogen wird, kuppelt man auf Phenol und filtriert den erhaltenen Farbstoff. Ausbeute an Farbstoff : 270 g, entsprechend 85 % der Theorie.

Beispiel 9

Im Vorratsgefäss 1 werden 2 080 ml einer 1,5 molaren Lösung von 1-Amino-2-sulfo-4-(4'-aminophenyl)aminoanthrachinon in 100 %-iger Schwefelsäure vorgelegt. Im Vorratsgefäss 3 legt man 277,6 ml 66 %-iges Oleum vor. Die Kühlschlaufe wird mit 2 200 ml Benzin mit einem Siedebereich von 110°-140 °C gefüllt. Man setzt die Zentrifugalpumpe 5 in Betrieb und bringt die Kühlmediumtemperaturen der Wärmeaustauscher auf folgende Werte : Wärmeaustauscher 9 : 10 °C, Wärmeaustauscher 9a : Raumtemperaur. Dann werden die beiden Reaktanden sofort mit den Dosierpumpen 2 und 4 in den Reaktor eindosiert. Der Durchsatz an 66 %-igem Oleum beträgt 13,88 ml ( = 0,075 Mol)/Min., jener an 1-Amino-2-sulfo-4-(4'-aminophenyl)aminoanthrachinon in Schwefelsäure 104 ml ( = 0,075 Mol)/Min. Nach ca. 2 Minuten stellen sich stationäre Bedingungen ein. Eine erwünschte Reaktionstemperatur von 30 °C ist dann erreicht. Eine zu hohe oder zu tiefe Reaktionstemperatur lässt sich problemlos über die Kühlung des Wärmeaustauschers 9 regulieren. Der Versuch dauert 20 Minuten. Die erhaltene wässrige Sulfiermasse wird aus dem Abscheider 7 abgezogen und auf Eis/Wasser ausgetragen. Der ausgefallene Feststoff wird filtriert. Man erhält so 660,2 g 1-Amino-2-sulfo-(2'-sulfo-4'-aminophenyl)aminoanthrachinon, entsprechend einer Ausbeute von 90 % der Theorie.

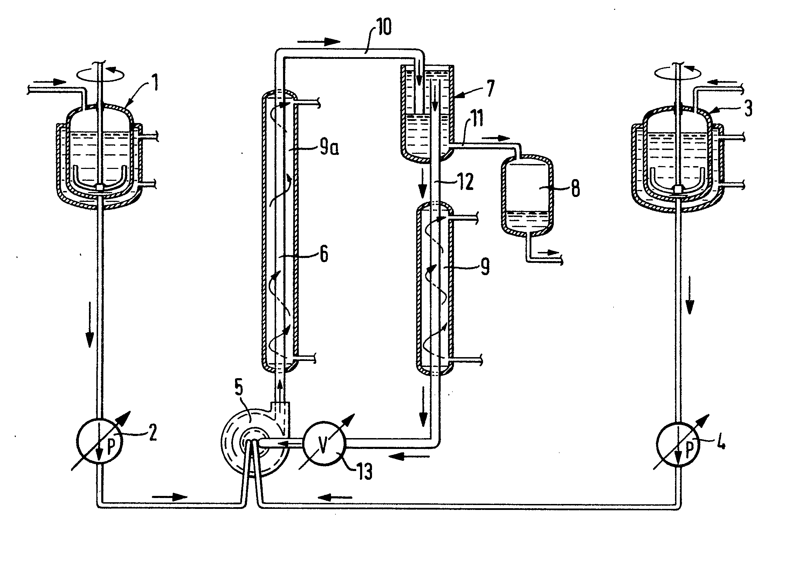

**Ansprüche**

1. Verfahren zur Abführung der Reaktionswärme von in flüssiger Phase ablaufenden, kontinuierlich geführten, exothermen chemischen Reaktionen, dadurch gekennzeichnet, dass man eine gegenüber den Reaktionspartnern inerte, diese und die Reaktionsprodukte vorzugsweise nicht oder nur wenig lösende, mit dem Reaktionsmedium oder Teilen des Reaktionsmediums nicht mischbare Flüssigkeit in einen das Reaktionsmedium enthaltenden Reaktor einleitet, worin eine innige Durchmischung erfolgt, die entstandene Emulsion in die die Reaktionsprodukte enthaltende Phase und die erwärmte Inertflüssigkeit auftrennt, letztere abkühlt und wieder dem Reaktor zuführt, wobei die Inertflüssigkeit diesen Kreislauf beliebig oft durchlaufen kann.

2. Verfahren nach Anspruch 1, dadurch gekennzeichnet, dass man als Reaktor eine Zentrifugalpumpe, einen On-line-Mischer mit Förderpumpe, einen statischen Mischer mit Förderpumpe, eine Mischturbine oder einen Strahldüsenreaktor einsetzt.

3. Verfahren nach Anspruch 1 oder 2, dadurch gekennzeichnet, dass man die aus dem Reaktor austretende Emulsion in ein gegebenenfalls mit einem Wärmeaustauscher versehenes Reaktionsrohr einbringt, bevor die Entmischung stattfindet, um eine Vervollständigung der Reaktion zu ermöglichen.

4. Verfahren nach Anspruch 1, dadurch gekennzeichnet, dass man als Inertflüssigkeit im Falle von rein wässrigen Reaktionsmedien aliphatische, cycloaliphatische oder aromatische Kohlenwasserstoffe, halogenierte aliphatische oder aromatische Kohlenwasserstoffe, nicht mit Wasser mischbare Alkohole, Ketone, Ester oder Aether einsetzt.

5. Verfahren nach Anspruch 1, dadurch gekennzeichnet, dass die Wärmeabführung aus exothermen Substitutions-, Oxidations-, Reduktions-, Additions-, Eliminierungs- oder Polymerisationsreaktionen erfolgt.

6. Verfahren nach Anspruch 5 dadurch gekennzeichnet, dass die Wärmeabführung aus Nitrierungs-, Sulfonierungs-, Diazotierungs-, Hydrierungs- und anderen Reduktionsreaktionen erfolgt.

7. Verfahren nach Anspruch 6, dadurch gekennzeichnet, dass die Wärmeabführung bei der Nitrierung aromatischer Systeme erfolgt.

8. Vorrichtung zur Durchführung des in den Ansprüchen 1 bis 7 beschriebenen Verfahrens, gekennzeichnet durch

a) einen Reaktor (5) mit Zuleitungen für die Ausgangsprodukte und gegebenenfalls für Lösungsmittel, und mit einer Zuleitung für die Inertflüssigkeit :

b) einen Abscheider (7), der durch eine Leitung (10) mit dem Reaktor verbunden ist, und in dem die Produktelösung von der Inertflüssigkeit abgetrennt wird und

c) eine mit einem Wärmeaustauscher (9) versehene Rückführungsleitung (12), die den Abscheider (7) mit dem Reaktor (5) verbindet.

9. Vorrichtung nach Anspruch 8, dadurch gekennzeichnet, dass der Reaktor (5) eine Zentrifugalpumpe, ein On-line-Mischer mit Förderpumpe, ein statischer Mischer mit Förderpumpe, eine Mischturbine oder ein Strahldüsenreaktor ist.

10. Vorrichtung nach Anspruch 8, dadurch gekennzeichnet, dass zwischen dem Reaktor (5) und dem Abscheider (7) eine Nachreaktionszone eingeschaltet ist.

11. Vorrichtung nach Anspruch 10, dadurch gekennzeichnet, dass ein Teil der Leitung zwischen dem Reaktor (5) und dem Abscheider (7) als Nachreaktionsrohr (6) ausgebildet ist.

12. Vorrichtung nach Anspruch 11, dadurch gekennzeichnet, dass das Nachreaktionsrohr (6) mit einem Wärmeaustauscher (9a) versehen ist.

## Claims

1. A method of removing the heat of reaction from continuous exothermic chemical reactions conducted in the liquid phase, which method comprises introducing a fluid which is inert to the reactants and in which reactants and reaction products are preferably insoluble or almost insoluble, and which is immiscible with the reaction medium or with parts thereof, into a reactor containing the reaction medium and wherein a homogeneous mixing is effected, separating the emulsion thereby obtained into the phase containing the reaction products and that consisting of the heated inert fluid, cooling said inert fluid and recycling it to the reactor, such that the inert fluid is able to pass through this circuit as often as desired.

2. A method according to claim 1, wherein the reactor is a centrifugal pump, an on-line mixer with feed pump, a static mixer with feed pump, a turbine mixer, or a jet tube reactor.

3. A method according to either of claims 1 or 2, wherein the emulsion issuing from the reactor is passed into a reaction pipe optionally provided with a heat exchanger before phase separation takes place, in order to bring the reaction to completion.

4. A method according to claim 1, wherein the inert fluid used for cooling purely aqueous reaction media is an aliphatic, cycloaliphatic or aromatic hydrocarbon, a halogenated aliphatic or aromatic hydrocarbon, a water-immiscible alcohol, a ketone, an ester or an ether.

5. A method according to claim 1, wherein the reaction from which heat is removed is an exothermic substitution, oxidation, reduction, addition, elimination or polymerisation reaction.

6. A method according to claim 5, wherein the reaction from which heat is removed is a nitration, sulfonation, diazotisation, hydrogenation or other reduction reaction.

7. A method according to claim 6, wherein heat of reaction is removed from the nitration of aromatic systems.

8. An assembly for carrying out the method of claims 1 to 7 comprising

a) a reactor (5) with feed lines for the starting materials and, if required, for the solvent or solvents, and with a feed line for a fluid which is inert to the reactants,

b) a separator (7) which is connected to the reactor by a line (10) and in which the solution containing the products is separated from the inert fluid, and

c) a return line (12) provided with a heat exhanger (9), which line connects the separator (7) to the reactor (5).

9. An assembly according to claim 8, wherein the reactor (5) is a centrifugal pump, an on-line mixer with feed pump, a static mixer with feed pump, a turbine mixer, or a jet tube mixer.

10. An assembly according to claim 8, wherein a further reaction zone is provided between the reactor (5) and the separator (7).

11. An assembly according to claim 10, wherein a part of the line between the reactor (5) and the separator (7) is a reaction pipe (6).

12. An assembly according to claim 11, wherein the reaction pipe (6) is provided with a heat exchanger (9a).

## Revendications

1. Procédé pour éliminer la chaleur de réaction de réactions chimiques exothermiques, conduites en continu, se déroulant en phase aqueuse, caractérisé en ce que l'on introduit un liquide inerte vis-à-vis des partenaires réactionnels, ne dissolvant pas, ou seulement peu, ces derniers et les produits de réaction, non miscibles au milieu réactionnel ou à des parties du milieu réactionnel, dans un réacteur contenant le milieu réactionnel, réacteur dans lequel s'effectue un mélangeage intime, on sépare l'émulsion obtenue en la phase contenant les produits de réaction et le liquide inerte chauffé, on refroidit ce dernier et on le renvoie au réacteur, le liquide inerte pouvant parcourir ce cycle aussi souvent qu'on le désire.

2. Procédé selon la revendication 1, caractérisé en ce que l'on utilise en tant que réacteur une pompe centrifuge, un mélangeur en ligne avec pompe de refoulement, un mélangeur statique avec pompe de refoulement, une turbine mélangeuse ou un réacteur à éjecteurs.

3. Procédé selon la revendication 1 ou 2, caractérisé en ce que l'on introduit l'émulsion sortant du réacteur dans un tube de réaction éventuellement muni d'un échangeur de chaleur avant que ne se produise la démixion, pour permettre un achèvement de la réaction.

4. Procédé selon la revendication 1, caractérisé en ce que l'on utilise en tant que liquide inerte, dans le cas de milieux réactionnels purement aqueux, des hydrocarbures aliphatiques, cycloaliphatiques ou aromatiques, des hydrocarbures aromatiques ou aliphatiques halogénés, des cétones, des esters, des éthers ou des alcools non miscibles à l'eau.

5. Procédé selon la revendication 1, caractérisé en ce que l'élimination de la chaleur s'effectue à partir de réactions exothermiques de substitution, d'oxydation, de réduction, d'addition, d'élimination ou de polymérisation.

6. Procédé selon la revendication 5, caractérisé en ce que l'élimination de la chaleur s'effectue à partir de réactions de nitration, de sulfonation, de diazotation, d'hydrogénation et d'autres réactions de réduction.

7. Procédé selon la revendication 6, caractérisé en ce que l'élimination de la chaleur s'effectue lors de la nitration de systèmes aromatiques.

8. Dispositif pour la mise en œuvre du procédé décrit dans les revendications 1 à 7, caractérisé par

a) un réacteur (5) avec des conduites d'amenée pour les produits de départ et éventuellement pour des solvants, et avec une conduite d'amenée pour le liquide inerte ;

b) un séparateur (7), qui est relié par une conduite (10) au réacteur, et dans lequel la solution des produits est séparée du liquide inerte et

c) une conduite de retour (12), garnie d'un échangeur de chaleur (9), et qui relie le séparateur (7) au réacteur (5).

9. Dispositif selon la revendication 8, caractérisé en ce que le réacteur (5) est une pompe centrifuge, un mélangeur en ligne avec pompe de refoulement, un mélangeur statique avec pompe de refoulement, une turbine mélangeuse ou un réacteur à éjecteurs.

10. Dispositif selon la revendication 8, caractérisé en ce qu'entre le réacteur (5) et le séparateur (7) est insérée une zone de post-réaction.

11. Dispositif selon la revendication 10, caractérisé en ce qu'une partie de la conduite entre le réacteur (5) et le séparateur (7) est conçue comme un tube de post-réaction (6).

12. Dispositif selon la revendication 11, caractérisé en ce que le tube de post-réaction (6) est muni d'un échangeur de chaleur (9a).

0 028 203